# EUROPÄISCHE PATENTANMELDUNG

(11) **EP 4 732 868 A1**
(43) Veröffentlichungstag der Anmeldung: **29.04.2026**
(21) Anmeldenummer: 24208692.4
(22) Anmeldetag: 24.10.2024
(51) Int. Cl.: A61M 5/168, A61M 5/14, A61M 5/172, A61M 5/36, A61M 5/142, A61M 5/145

(54) **MEDIZINISCHE PUMPEINRICHTUNG UND INFUSIONSSYSTEM MIT EINER SOLCHEN PUMPEINRICHTUNG**

(71) Anmelder: ConnCons GmbH, 01307 Dresden (DE)
(72) Erfinder: Gommel, Christoph, 01445 Radebeul (DE); Hampe, Jochen, 01277 Dresden (DE); Schurig, Carsten, 01445 Radebeul (DE); Curran, Joseph, 01239 Dresden (DE); Engler, Katharina, 01099 Dresden (DE); Dietze, Carolin, 01309 Dresden (DE)
(74) Vertreter: Witte, Weller und Partner Patentanwälte mbB Stuttgart

(57) **Zusammenfassung**

Vorgeschlagen wird eine medizinische Pumpeinrichtung (10) mit einer Basiseinheit.

Diese Pumpeinrichtung (10) verfügt über mindestens zwei flüssigkeitsleitende Wechselmodule (50A, 50B, 50C), die an einem Aufnahmebereich (22) der Basiseinheit (12) gleichzeitig ankoppelbar sind. Jedes der flüssigkeitsleitenden Wechselmodule (50A, 50B, 50C) weist mindestens einen Flüssigkeitseingang (52) und mindestens einen Flüssigkeitsausgang (54) sowie einen vom mindestens einen Flüssigkeitseingang (52) zum Flüssigkeitsausgang (54) führenden Flüssigkeitskanal (60) auf, wobei im Flüssigkeitskanal (60) mindestens eine Pumpkammer (72) oder mindestens ein Pumpabschnitt vorgesehen ist.

## Beschreibung

### ANWENDUNGSGEBIET UND STAND DER TECHNIK

Die Erfindung betrifft eine medizinische Pumpeinrichtung, insbesondere vorgesehen für die Verwendung eines Infusionssystems. Die Erfindung betrifft darüber hinaus auch das Infusionssystem als Ganzes.

Infusionssysteme dienen der kontrollierten Verabreichung von Flüssigkeiten, Medikamenten oder Nährstoffen in den Körper eines Patienten. Infusionssysteme verwenden in der Regel Infusionspumpen, um eine präzise und gleichmäßige Abgabe zu gewährleisten. Moderne Infusionspumpen bieten dabei Funktionen zur Überwachung und Alarmierung bei Unregelmäßigkeiten, um die Sicherheit des Patienten zu gewährleisten. Sie finden in der Medizin, insbesondere in der Intensivpflege und bei Langzeittherapien, Anwendung.

Eine gattungsgemäße Pumpeinrichtung verfügt über eine Basiseinheit sowie ein hieran wechselbar ankoppelbares Wechselmodul. Dies gestattet es, die flüssigkeitsführenden Flächen allein im Wechselmodul vorzusehen und die Basiseinheit mit Aktoren und Antrieben zu versehen, die selbst nicht in Flüssigkeitskontakt gelangen.

Im Rahmen von Infusionstherapien gibt es je nach konkretem Falle unterschiedliche Bedürfnisse, was die Zahl der zu verabreichenden Flüssigkeiten und die gemeinsame oder getrennte Verabreichung angeht. Bekannte medizinische Pumpeinrichtungen weisen jedoch keine hohe Flexibilität auf, so dass unterschiedliche Anforderungen dazu führen können, dass bestimmte Pumpeinrichtungen im Einzelfall nicht nutzbarsind.

### AUFGABE UND LÖSUNG

Aufgabe der Erfindung ist es, eine besonders flexibel einsetzbare medizinische Pumpeinrichtung zur Verfügung zu stellen.

Erfindungsgemäß wird zur Lösung dieser Aufgabe eine medizinische Pumpeinrichtung vorgeschlagen sowie ein Infusionssystem mit einer solchen Pumpeinrichtung.

Eine Pumpeinrichtung erfindungsgemäßer Art ist in besonderer Weise modular ausgebildet und verfügt über eine Basiseinheit sowie Wechselmodule.

Die Basiseinheit bildet dabei üblicherweise den Hauptbestandteil der Pumpeinrichtung. Die Basiseinheit weist üblicherweise ein Gehäuse auf, welches verschiedene Steuer- und Antriebsfunktionen beherbergen kann. An einer Seite, üblicherweise bei bestimmungsgemäßer Anordnung der Basiseinheit an einer Vorderseite, ist ein Aufnahmebereich für die Wechselmodule vorgesehen. Hier können Wechselmodule in verschiedenen Konfigurationen angebracht werden. Der Betrieb dieser Wechselmodule kann dann durch verschiedene Aktoren oder Motoren gewährleistet werden, die im Aufnahmebereich auf der Seite der Basiseinheit vorgesehen sind und mit korrespondierenden Komponenten der Wechselmodule zusammenwirken.

Die Basiseinheit ist dafür ausgebildet, mindestens zwei Wechselmodule derart aufzunehmen, dass beide Wechselmodule verwendbar sind, also die Pumpe im installierten Zustand in Betrieb gesetzt werden kann und Flüssigkeit von den jeweiligen Flüssigkeitseingängen zum jeweiligen Flüssigkeitsausgang zu fördern. Die Konfiguration der Basiseinheit kann über Basismodule der Basiseinheit, die im Weiteren noch erläutert werden, anpassbar gestaltet sein.

Die primäre erfindungsgemäße Besonderheit der Pumpeinrichtung besteht darin, dass die Basiseinheit eine Mehrzahl von Wechselmodulen aufnehmen kann. Dies gestattet es, fallweise unterschiedliche Konfigurationen zu verwenden, die jeweils an den betreffenden Patienten und dessen Medikation angepasst sind und einfach geändert werden können.

Üblicherweise verfügen die zur Ankopplung an die Basiseinheit vorgesehenen Wechselmodule über eine Mehrzahl von Flüssigkeitseingängen, typischerweise über mindestens zwei Flüssigkeitseingänge. Vorzugsweise stehen unterschiedliche Arten von Wechselmodulen zur Verfügung, die sich insbesondere bezüglich der Zahl der Flüssigkeitseingänge unterscheiden. Trotz der Tatsache, dass die Basiseinheit zur Aufnahme von mindestens zwei Wechselmodulen ausgestaltet ist, ist vorzugsweise auch der Betrieb mit nur einem Wechselmodul möglich.

Die üblicherweise mindestens zwei Flüssigkeitseingänge und der üblicherweise eine Flüssigkeitsausgang sind über einen Flüssigkeitskanal miteinander verbunden, wobei hierunter im Kontext dieser Erfindung alle flüssigkeitsführenden Kammern des Wechselmoduls zu verstehen sind. Der Flüssigkeitskanal weist üblicherweise mindestens eine Pumpkammer auf, die zyklisch verkleinerbar und vergrößerbar ist, um Flüssigkeit zu fördern. Alternativ zur Pumpkammer kann auch ein Pumpabschnitt vorgesehen sein, innerhalb dessen die Flüssigkeit Richtung Flüssigkeitsausgang gefördert wird. Der Pumpabschnitt ist in diesem Falle also Teil einer Schlauchpumpe.

Im Flüssigkeitskanal können weiterhin auch Filter vorgesehen sein, so insbesondere ein Sterilfilter oder ein Partikelfilter mit einer Trenngrenze von vorzugsweise 0,2µm oder weniger.

Weiterhin kann im Flüssigkeitskanal auch eine Air-Trap-Vorrichtung vorgesehen sein. Diese stellt sicher, dass keine Luftblasen am Flüssigkeitsausgang mit abgegeben werden. Die Air-Trap besteht vorzugsweise aus einer Kammer, die stromaufwärts der Pumpkammer bzw. des Pumpabschnitts angeordnet ist und dazu ausgelegt ist, Luftblasen aus der Flüssigkeit zu entfernen. In der Kammer kann insbesondere ein hydrophober Filter angeordnet sein, der Flüssigkeiten durchlässt,jedoch Luft zurückhält.

Im einfachsten Falle kann die Basiseinheit Platz für zwei baugleiche Wechselmodule zur Verfügung stellen, jedoch bei entsprechendem Bedarf nur mit einem dieser Wechselmodule betrieben werden, während der andere Aufnahmeplatz frei bleibt. In anderen Fällen kann die Basiseinheit Platz für Wechselmodule unterschiedlicher Bauart und insbesondere unterschiedlicher Anzahl von Flüssigkeitseingängen aufweisen, so dass bedarfsgerecht beispielsweise eine Anzahl von Flüssigkeiten für einen Patienten auf mehrere Wechselmodule verteilt wird, von denen eines für wenige, beispielsweise zwei, zugeführte Flüssigkeiten vorgesehen ist, während das andere für mehr, beispielsweise drei oder vier Flüssigkeiten vorgesehen ist.

Die Pumpbetätigung des Pumpabschnitts oder der Pumpkammer in der Flüssigkeitsleitung und damit die Flüssigkeitsförderung von mindestens einem Flüssigkeitseingang zum zugehörigen Flüssigkeitsausgang kann mittels eines in das Wechselmodul integrierten Pumpenmotors als Pumpenantrieb erfolgen. Bevorzugt ist es jedoch, wenn der Pumpenantrieb auf Seiten der Basiseinheit vorgesehen ist.

Bevorzugt ist eine Bauweise, bei der der mindestens eine Pumpenantrieb als pneumatischer Pumpenantrieb ausgebildet ist. Dies bedeutet, dass mittels eines Elektromotors im Wechsel eine Druckerhöhung und eine Druckverringerung erzeugt wird, die an einem Druckanschluss im Aufnahmebereich der Basiseinheit anliegt. Dies erzeugt alternierend eine Vergrößerung und eine Verkleinerung in der Pumpkammer des Wechselmoduls.

Vorzugsweise weist die Basiseinheit mehrere getrennte Pumpenantriebe auf, die jeweils einem Wechselmodul zugeordnet sind. Insbesondere vorzugsweise sind je nach Konfiguration von Wechselmodulen nur ein Teil der Pumpenantriebe in Betrieb. Beispielsweise kann vorgesehen sein, dass an der Basiseinheit drei oder mehr getrennt betreibbare Pumpenantriebe vorgesehen sind, obwohl in der Praxis meist nur zwei Wechselmodule verwendet werden. Da aber je nach Konfiguration von Wechselmodulen deren Pumpkammern oder Pumpabschnitte an verschiedenen Stellen relativ zum Aufnahmebereich angeordnet sind, kann die Bereitstellung von drei oder mehr Pumpenantrieben zweckmäßig sein, so dass für alle möglichen Wechselmodulkonfigurationen die passenden Pumpenantriebe bereitstehen. Die Pumpenantriebe sind vorzugsweise an unterschiedlichen Basismodulen der Basiseinheit vorgesehen.

Vorzugsweise sind die Wechselmodule auch dafür ausgebildet, den Flüssigkeitsstrom von einem einzelnen Flüssigkeitseingang oder zum Flüssigkeitsausgang unterbrechen zu können und verfügt hierfür über eine Ventileinrichtung in ihrem Flüssigkeitskanal. Eine Ventileinrichtung im Sinne dieser Beschreibung weist gegeneinander verlagerbare Flächen im Flüssigkeitskontakt auf, die in eine Schließstellung der Flächen den Flüssigkeitskanal versperren.

Ein Ventilaktor, um die genannte Bewegung durchzuführen, kann Teil des jeweiligen Wechselmoduls sein. Bevorzugt ist es allerdings, wenn an der Basiseinheit mindestens ein Ventilaktor vorgesehen ist, mittels dessen die mindestens eine Ventileinrichtung des Wechselmoduls geschlossen und geöffnet werden kann. Ein solcher Ventilaktor kann beispielsweise einen am Aufnahmebereich ausfahrbaren Aktorstift umfassen, der durch Ausfahren und Einfahren auf eine Gegenfläche der Ventileinrichtung wirkt und diese öffnet oder schließt.

Weiterhin ist es auch möglich, dass das Wechselmodul über eine Einrichtung zur Flüssigkeitsmengensteuerung verfügt. Eine solche Einrichtung gestattet eine besonders feine oder genaue Dosierung, ohne dass die Dosierung allein durch die Pumpkammer oder den Pumpabschnitt geregelt werden muss. Insbesondere kann die Flüssigkeitsmengensteuerung ein variabel einstellbares Steuerventil umfassen, wobei insbesondere zur Regelung der Flüssigkeitsmengensteuerung die Basiseinheit einen Stellmotor aufweist, der vorzugsweise als Schrittmotor gestaltet ist und der vorzugsweise auf das genannte Stellventil wirkt.

Grundsätzlich ist eine Bauweise denkbar und je nach Anforderungsprofil auch angemessen, bei der die Wechselmodule keinerlei elektrische oder bestimmungsgemäß elektrizitätsleitende Komponenten umfasst, sondern rein mechanisch ausgebildet ist. In diesem Falle sind alle erforderlichen Aktoren auf Seiten der Basiseinheit vorgesehen.

Es kann jedoch auch verschiedene Gründe geben, hiervon abweichend die Wechselmodule mit Daten- und/oder Stromkonnektoren zu versehen und hierüber eine Verbindung mit der Basiseinheit herzustellen. Die Basiseinheit verfügt zu diesem Zweck im Aufnahmebereich über mindestens zwei entsprechende Daten- und/oder Stromkonnektoren. Die Daten- und/oder Stromkonnektoren verfügen vorzugsweise über frei liegende Kontaktflächen, um eine unmittelbare galvanische Kopplung der Basiseinheit mit den Wechselmodulen zu ermöglichen. Auch andere Übertragungsmöglichkeiten wie Induktion und Kapazität sind möglich.

Mittels der Datenkonnektoren können elektrische Signale oder elektrische Leistung zwischen der Basiseinheit und den mindestens zwei Wechselmodulen ausgetauscht werden.

Das Wechselmodul kann zu diesem Zweck einen integrierten Schaltkreis aufweisen. Dieser kann einen Speicher umfassen, der Typinformationen über das Wechselmodul und/oder eine eindeutige Kennzeichnung enthalten kann, die über die Datenkonnektoren an die Basiseinheit übertragen werden kann. Weitere im Speicher vorzugsweise hinterlegte Daten betreffen Spezifikationen des Wechselmoduls, insbesondere Daten zu den zur Ansteuerung der Funktionen über Aktoren und Motoren der Basiseinheit, über Kompatibilitäten sowie Daten zu Fluid-Volumina des spezifischen Wechselmoduls. Auch Daten zur Benutzungshistorie oder Haltbarkeitsdaten können hier hinterlegt sein. Der Datenaustausch über die Datenkonnektoren kann weiterhin dem Zweck dienen, Aktoren für die Steuerung des Wechselmoduls unmittelbar in das Wechselmodul zu integrieren. Auch sind Gestaltungen hierdurch möglich, bei denen mindestens ein Wechselmodul mindestens einen Sensor aufweist, der mit dem Datenkonnektor des Wechselmoduls verbunden ist, so dass die Ausgangsdaten des Sensors durch eine nicht in das Wechselmodul integrierte Elektronik ausgewertet werden kann, beispielsweise eine Elektronik der Basiseinheit. Entsprechende Sensoren können vor allem Drucksensoren, Luftblasendetektoren, Durchflusssensoren oder Temperatursensoren sein.

Insbesondere kann die Verbindung zwischen der Basiseinheit und den Wechselmodulen aber dem Zweck dienen, hierüber eine Datenverbindung mit an den Wechselmodulen angeschlossenen Geräten und Vorrichtungen zu ermöglichen. So kann beispielsweise das Auslesen von Daten von einem intelligenten Medikationsbeutel über die genannten Datenkonnektoren erfolgen.

Hierfür kann insbesondere vorgesehen sein, dass der mindestens eine Datenkonnektor eines Wechselmoduls mit einem in den Flüssigkeitseingang und/oder in den Flüssigkeitsausgang integrierten weiteren Datenkonnektor über Signalleitungen verbunden ist. Die hier angeschlossenen Schläuche sind ebenfalls für den Datentransfer ausgestaltet und verfügen daher vorzugsweise ebenfalls über einen in einen Flüssigkeitskonnektor integrierten Datenkonnektor.

Neben einer Gestaltung, bei der Sensoren an den Wechselmodulen vorgesehen sind, kann auch eine Gestaltung vorteilhaft sein, bei der an der Basiseinheit mindestens ein Sensor vorgesehen ist, der Sensordaten erzeugt. Dieser Sensor, der insbesondere ein Luftblasendetektor sein kann, erzeugt Sensordaten durch eine Datenerfassung am Flüssigkeitskanal des im Sensorbereich angebrachten Wechselmoduls. Insbesondere kann der Sensor als optischer Sensor ausgebildet sein, der in Richtung des angekoppelten Wechselmoduls ausgerichtet ist, wobei vorzugsweise am Wechselmodul eine Aussparung oder ein Sichtfenster im Bereich des optischen Sensors vorgesehen ist.

Grundsätzlich ist eine Bauweise möglich, bei der die Kopplung von Wechselmodulen an die Basiseinheit bewusst erschwert ist, beispielsweise durch eine Verschraubung. Eine solche Kopplung kann entsprechend nur mit vergleichsweise großem Aufwand gelöst werden, ist aber gleichzeitig von Vorteil, da eine ungewollte Trennung eines Wechselmoduls von der Basiseinheit nicht droht.

Bevorzugt ist es allerdings, dass die Basiseinheit und die Wechselmodule für eine werkzeuglose Kopplung und Entkopplung ausgebildet sind. Hierfür können beispielsweise die Basiseinheit und die Wechselmodule in einem oberen Bereich des Aufnahmebereichs der Basiseinheit und in einem oberen Bereich einer Rückwandung der Wechselmodule zusammenwirkende Hängegeometrien aufweisen.

Alternativ oder ergänzend können die Basiseinheit und die Wechselmodule zusammenwirkende Schnappgeometrien aufweisen, mittels derer die Wechselmodule an der Basiseinheit festschnappbar sind.

Ebenfalls zweckmäßig kann die Verwendung von Magneten sein, um die Wechselmodule im Aufnahmebereich zu befestigen. Bei einer solchen Konfiguration sind am Aufnahmebereich und an den Wechselmodulen zusammenwirkende Magneten bzw. magnetisierbare Flächen vorgesehen.

Eine besonders vorteilhafte Gestaltung sieht eine Kombination aus Hängegeometrien und Magneten vor. Die Wechselmodule sind dabei derart an die Basiseinheit angepasst, dass sie zunächst mittels korrespondierender Hängegeometrien eingehängt werden und dann in ihre Endstellung verschwenkt werden, wo sie dann zusätzlich magnetisch gehalten sind. Während des Schwenkens wird ggf. gleichzeitig die mechanische Kopplung zwischen den Wechselmodulen und den Aktoren auf Seite der Basiseinheit hergestellt.

Die Basiseinheit, an der in oben beschriebener Weise bspw. Pumpenantriebe und Ventilaktoren vorgesehen sind, die auf die angekoppelten Wechselmodule wirken, kann auch als solche ebenfalls modular ausgestaltet sein.

Bevorzugt ist eine Bauweise, bei der die Basiseinheit eine Mehrzahl von Basismodulen aufweist, die separat austauschbar sind und deren Vorderseite jeweils den Aufnahmebereich zur Aufnahme der Wechselmodule begrenzt. Diese Basismodule werden üblicherweise fest an einem Grundkörper der Basiseinheit befestigt, insbesondere dort verschraubt.

Die Basismodule umfassen üblicherweise einen Pumpenantriebe der oben beschriebenen Art und/oder mindestens einen Ventilaktor der oben beschriebenen Art. Üblicherweise ist ein Basismodul somit geeignet, ein Wechselmodul zu steuern. Basismodule können weiterhin auch Sensoren umfassen zur Erfassung des Zustandes des Wechselmoduls. Es können auch Basismodule vorgesehen sein, die zur Steuerung mehrerer Wechselmodule vorgesehen sind und zu diesem Zweck bspw. mehrere Pumpenantriebe oder entsprechende Ventilaktoren und/oder Sensoren umfassen.

Die modulare Gestaltung mit Basismodulen gestattet es, die Basiseinheit für unterschiedliche im Betrieb mögliche Konfigurationen auszugestalten. Während die Anpassung der Pumpeinrichtung durch Wechselmodule im täglichen Betrieb vorgesehen ist, ist die Konfigurierbarkeit der Basiseinheit zur Herstellung einer üblicherweise dauerhaften Konfiguration vorgesehen.

Zwar sind auch Bauweisen denkbar, bei denen auch die Basiseinheit eine flüssigkeitsführende Funktion aufweist. Dies ist jedoch in der Regel nicht gewollt. Stattdessen wird eine Gestaltung bevorzugt, bei der die Basiseinheit keinerlei flüssigkeitsführende Kanäle, Kammern oder Flächen aufweist.

Die Erfindung betrifft weiterhin auch ein Infusionssystem, bei dem eine Pumpeinrichtung der beschriebenen Art Verwendung findet. Das Infusionssystem umfasst mindestens eine solche Pumpeinrichtung zur Förderung von medizinischen Flüssigkeiten aus Medikationsbehältern zu einem Patienten.

Obwohl der Schwerpunkt der Erfindung auf eine Pumpeinrichtung liegt, an der eine Mehrzahl von Wechselmodulen der beschriebenen Art ankoppelbar sind, werden auch die Ausgestaltung der einzelnen Wechselmodule in der oben beschriebenen Art als Teil der Erfindung angesehen, auch im Kontext einer Pumpeinrichtung, die nur zur Aufnahme eines solchen Wechselmoduls vorgesehen ist und mehrere Wechselmodule nicht aufnehmen kann.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt ein Infusionssystem, das einem Patienten fünf verschiedene Flüssigkeiten über einen Venenkatheter zuführt.
Fig. 2 zeigt eine Detailansicht der Pumpeinrichtung im geschlossenen Zustand mit Flüssigkeitseingängen, Flüssigkeitsausgängen und einem Display an der Tür.
Fig. 3 zeigt die Pumpeinrichtung bei geöffneter Tür, wobei die beiden Wechselmodule im Aufnahmebereich sichtbar sind.
Fig. 4A und 4B zeigen zwei verschiedene Grundkonfigurationen der Basiseinheit der Pumpeinrichtung mit unterschiedlichen Basismodulen.
Fig. 5 zeigt drei verschiedene Wechselmodule zur Anpassung der Pumpeinrichtung an den jeweiligen Patienten und seine Behandlung.
Fig. 6 verdeutlicht den Aufbau der Basiseinheit der Pumpeinrichtung mit eingeschobenen Basismodulen.
Fig. 7A und 7B zeigen das Wechselmodul mit zwei Flüssigkeitseingängen und einem Flüssigkeitsausgang, sowohl von der Vorder- als auch von der Rückseite.
Fig. 8A bis 8C zeigen den werkzeuglosen Ankoppelvorgang der Wechselmodule an die Basiseinheit.

### DETAILLIERTE BESCHREIBUNG DER AUSFÜHRUNGSBEISPIELE

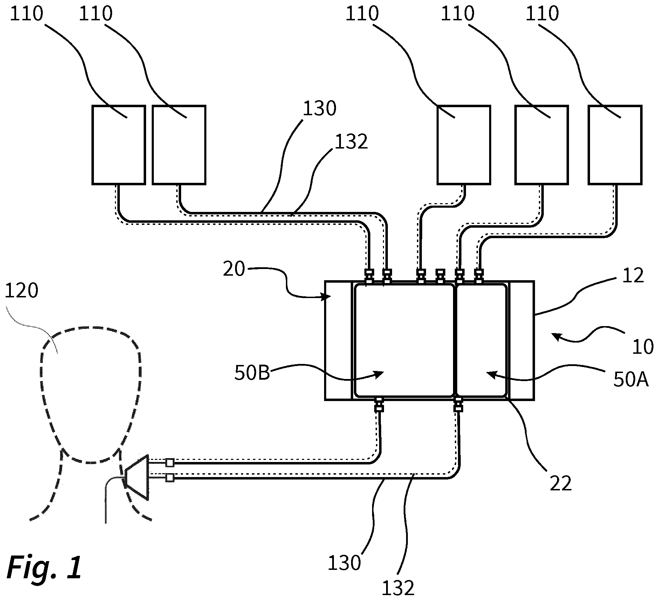

Fig. 1 zeigt ein erfindungsgemäßes Infusionssystem 100, welches der Versorgung eines Patienten 120 dient. Am Patienten 120 ist ein Venenkatheter 140 angeschlossen, um insgesamt fünf Flüssigkeiten zuführen zu können, die in Medikationsbeuteln 110 gelagert sind.

Das Infusionssystem 100 verfügt über eine Pumpeinrichtung 10, an der einerseits über fünf Flüssigkeitsleitungen 130 die fünf Medikationsbeutel 110 angeschlossen sind und andererseits über zwei Flüssigkeitsleitungen 130, an denen derVenenkatheter 140 angeschlossen ist.

Der Venenkatheter 140 und die Medikationsbeutel 110 sind nicht nur über die Flüssigkeitsleitungen 130 selbst, sondern auch über Datenleitungen 132 mit der Pumpeinrichtung 10 verbunden.

Die Pumpeinrichtung 10 selbst weist in der bereits aus Fig. 1 ersichtlichen Weise eine Basiseinheit 12 auf, die üblicherweise an einem ortsfesten oder rollbaren Infusionspumpenständer angebracht ist. Diese Basiseinheit 12 weist an ihrer Vorderseite einen Aufnahmebereich 22 auf, im Bereich dessen zwei Wechselmodule 50A, 50B vorgesehen sind. Die Wechselmodule 50A, 50B sind als Wechselkassetten ausgebildet, die ein Außengehäuse aufweisen, innerhalb dessen verschiedene Funktionselemente vorgesehen sind, die nachfolgend beschrieben werden. Das Wechselmodul 50A weist zwei Flüssigkeitseingänge auf, an denen zwei der Medikationsbeutel 110 angeschlossen sind. Das Wechselmodul 50B weist vier Flüssigkeitseingänge auf, an denen die drei anderen Medikationsbeutel 110 angeschlossen sind.

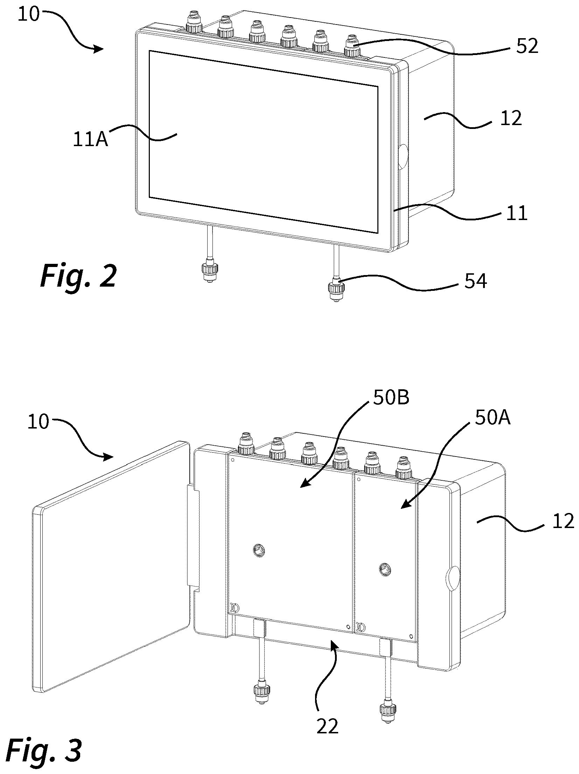

Fig. 2 zeigt die Pumpeinrichtung 10 in einer Detailansicht im geschlossenen Zustand. Wie bereits erläutert, verfügt die Pumpeinrichtung 10 über insgesamt sechs Flüssigkeitseingänge 52 und zwei Flüssigkeitsausgänge 54. Die Pumpeinrichtung 10 verfügt über eine Tür 11 mit einem integrierten Display 11A, auf dem Betriebsparameter angezeigt werden können.

Fig. 3 zeigt die Pumpeinrichtung 10 bei geöffneter Tür 11. Zu erkennen sind die beiden Wechselmodule 50A, 50B, die in den Aufnahmebereich 22 der Pumpeinrichtung 10 eingesetzt sind. Vier der Flüssigkeitseingänge 52 und einer der Flüssigkeitsausgänge 54 sind Teil des Wechselmoduls 50B. Die beiden anderen Flüssigkeitseingänge 52 und der zweite Flüssigkeitsausgang 54 sind Teil des Wechselmoduls 50A.

Die Wechselmodule 50A, 50B sind, wie im Weiteren noch genauer erläutert wird, dafür ausgebildet, Flüssigkeit von den Flüssigkeitseingängen 52 zu den Flüssigkeitsausgängen 54 zu fördern, wobei hierfür jedes Wechselmodul unter anderem eine Pumpkammer 72 und Ventileinrichtungen 86 aufweist, die von Seiten der Basiseinheit 12 gesteuert werden. Dies wird im Weiteren noch erläutert.

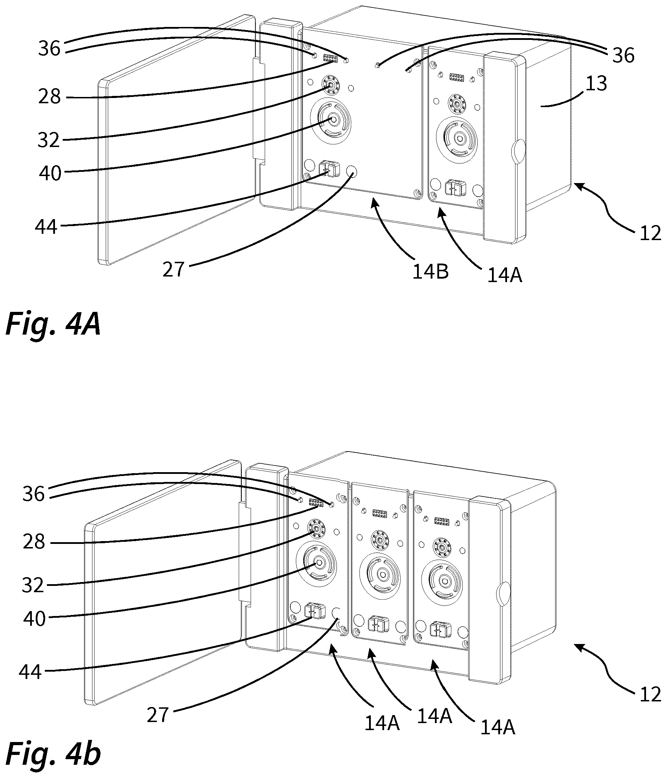

Die Fig. 4A und 4B zeigen die Basiseinheit 12 in zwei unterschiedlichen Grundkonfigurationen. Diese Grundkonfigurationen der Basiseinheit 12 unterschieden sich dadurch, welche Basismodule 14A, 14B hier in einem Grundkörper 13 der Basiseinheit 12 installiert sind.

Im Falle der Konfiguration der Fig. 3 verfügt die Basiseinheit 12 über zwei Basismodule 14A, 14B, wobei das Basismodul 14B doppelt so breit ist wie das Basismodul 14A. Übereinstimmend weisen beide Basismodule 14A, 14B jedoch jeweils die Komponenten auf, um ein Wechselmodul 50A, 50B ansteuern zu können.

Im Einzelnen verfügen beide Basismodule 14A, 14B jeweils über einen Druckanschluss 32, der von einem Pumpenantrieb 30 angetrieben wird, über eine Mehrzahl von Ventilaktoren 36 sowie über einen Stellmotor 40. Weiterhin ist an der Vorderseite der Basismodule 14A, 14B jeweils ein optischer Sensor 44 zur Erfassung von Luftbläschen vorgesehen. Voneinander unterscheiden sich die beiden Basismodule 14A, 14B vor allen Dingen dadurch, dass das Basismodul 14B über vier Ventilaktoren 36 für Steuerung von vier Flüssigkeitseingängen 52 verfügt, während das Basismodul 14A nur zwei solche Ventilaktoren umfasst. Weiterhin weisen die Basismodule 14A, 14B jeweils einen Daten- und/oder Stromkonnektor 28 auf, der zum Austausch von Daten oder elektrischer Leistung mit den Wechselmodulen 50A, 50B, 50C ausgebildet ist.

Die Grundkonfiguration der Fig. 4A gestattet die Ankoppelung von zwei Wechselmodulen 50A, 50B, wobei es sich je nach Bedarf und eine Kombination der Wechselmodule 50A, 50B handeln kann oder auch zwei Wechselmodule von der Art des Wechselmoduls 50A vorgesehen sein können. Auch ein einzeln betriebenes Wechselmodul kann angekoppelt werden.

Die Grundkonfiguration der Fig. 4B unterscheidet sich von der der Fig. 4A dadurch, dass hier drei baugleiche Basismodule 14A Verwendung finden. Somit weist die Basiseinheit 12 insgesamt drei Pumpenantriebe 30 auf.

Diese zweite Grundkonfiguration gestattet die gleichen Wechselmodulkonfigurationen wie die erste Grundkonfiguration der Fig. 2. Zusätzlich ist es hier jedoch möglich, drei Wechselmodule von derArt des Wechselmoduls 50A gleichzeitig und getrennt voneinander anzusteuern, da für jedes der drei Wechselmodule 50A ein eigener Pumpenantrieb 30 zur Verfügung steht.

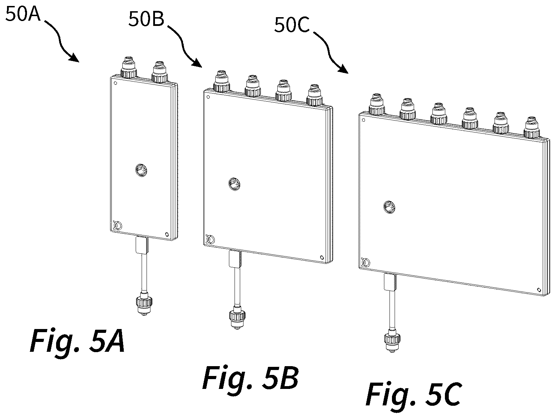

Fig. 5 zeigt mögliche Wechselmodule 50A, 50B, 50C zur Anpassung der Pumpeinrichtung 10 im täglichen Betrieb. Die Wechselmodule 50A, 50B, 50C unterscheiden sich primär bezüglich der Anzahl der Flüssigkeitseingänge 52. Entsprechend verfügen sie auch über eine unterschiedliche Anzahl von Ventileinrichtungen 86, von denen jeweils eine jedem Flüssigkeitseingang zugeordnet ist.

Die übrigen Funktionselemente der Wechselmodule sind bei allen drei Varianten in der gleichen Relativstellung zueinander vorgesehen. Trotz der unterschiedlichen Größe sind somit die gleichen korrespondierenden Aktoren/Motoren der Basiseinheit 12 geeignet, die unterschiedlichen Wechselmodule 50A, 50B, 50C zu betreiben.

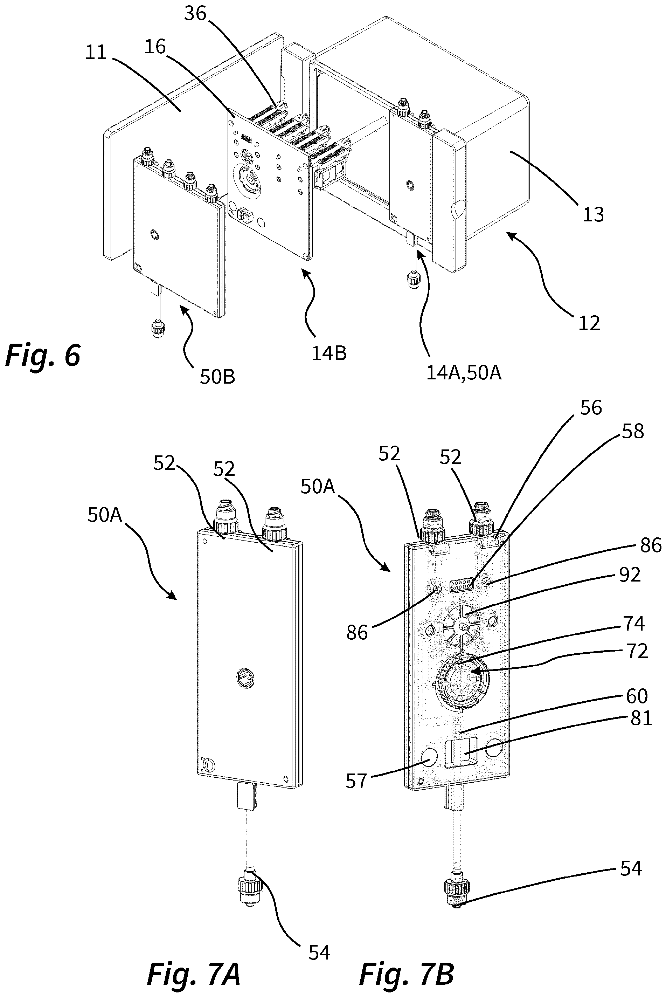

Fig. 6 verdeutlicht anhand der ersten Grundkonfiguration der Fig. 4A nochmals den modularen Charakter auch der Basiseinheit 12 selbst.

Wie zu ersehen ist, umfasst die Basiseinheit 12 einen Grundkörper 13, an dem die Tür 11 angebracht ist und derzurAufnahme der Basismodule 14A, 14B vorgesehen ist. Im dargestellten Zustand ist das Basismodul 14A fest eingesetzt und verschraubt und das Wechselmodul 50B ist daran werkzeuglos befestigt.

Das Basismodul 14B ist noch nicht eingesetzt. Es ist daher der Aufbau des Basismoduls 14B gut zu erkennen. Das Basismodul 14A weist eine vordere Blende 16 auf, an deren Rückseite verschiedene Aktoren angebracht sind und an deren Vorderseite der Sensor 44 zur Erkennung von Luftblasen vorgesehen ist. Die Blende 16, die eine Anlagefläche für die Wechselmodule 50A, 50B, 50C bildet, ist mit verschiedenen Durchbrüchen für die Aktoren versehen.

Das Basismodul 14B wird im Zuge der Herstellung der Basiseinheit 12 in den in Fig. 6 erkennbaren Schacht eingeschoben und mittels Schrauben gesichert. Es kann dann das Wechselmodul 50B aufnehmen.

Die Figuren 7A und 7B zeigen das Wechselmodul 50A mit zwei Flüssigkeitseingängen 52 und einem Flüssigkeitsausgang 54 in detaillierterer Darstellung. Figur 7A zeigt das Wechselmodul von der Vorderseite, Figur 7B von der Rückseite.

Die Wechselmodule 50A selbst weisen die bereits genannten zwei Flüssigkeitseingänge 52 auf, von denen aus sich ein Flüssigkeitskanal 60 in Richtung des Flüssigkeitsausgangs 54 erstreckt. Jedem Flüssigkeitseingang 52 ist eine Ventileinrichtung 86 zugeordnet, mittels derer der Zufluss aus den zwei Flüssigkeitseingänge 52 ermöglicht und unterbunden werden kann. Die durch die Flüssigkeitseingänge 52 einströmende Flüssigkeit wird mittels einer Pumpkammer 72 weitergefördert. Diese Pumpkammer 72 ist an der Rückseite der Wechselmodule durch eine Membranwandung 74 angeschlossen, die bei angebrachtem Modul im Bereich eines Druckanschlusses 32 liegt. Der dort aktivierbare Druckwechsel führt dann zu einer Verlagerung der Membranwandung 74 sowie damit zu einer zyklischen Änderung des Pumpkammervolumens. Zusammen mit nicht näher dargestellten Einlass- und Auslassventilen, insbesondere passiven Einlass- und Auslassventilen, führt diese zyklische Änderung zu einer Pumpwirkung in Richtung des Flüssigkeitsauslasses 54.

Im Flüssigkeitskanal 60 schließt sich an die Pumpkammer 72 eine Einrichtung 92 zur Flüssigkeitsmengensteuerung an, die über den korrespondierenden Aktor 40 der Basiseinheit 12 gesteuert werden kann.

Als letztes funktionales Element vor dem Flüssigkeitsauslass 54 folgt eine Aussparung 81 für den Sensor 44 der Basiseinheit 12, um Luftbläschenbildung zu erkennen, wie sie im Falle von fehlerhaften Fluidverbindungen oder bei einem leeren Medikationsspeicher auftreten können. Der entsprechende optische Sensor ist der bereits beschriebene basiseinheitsseitige Sensor 44, der in Richtung des angekoppelten Wechselmoduls ausgerichtet ist und somit die Flüssigkeit im Flüssigkeitskanal im Bereich der Aussparung 81 erfassen kann.

Zum Zwecke der Ankoppelung an die Basiseinheit 12 verfügt das Wechselmodule 50A an seinem oberen Ende über eine hakenartig ausgebildete Hängegeometrie 56. Am unteren Ende verfügt das Wechselmodul über Magneten 57 zur Befestigung an der Basiseinheit 12.

Korrespondierend sind an der Basiseinheit eine Hängegeometrie 26 sowie Magneten 27 vorgesehen. Die Anbringung der Wechselmodule 50A, 50B, 50C im Aufnahmebereich 22 erfolgt mittels dieser Hängegeometrien 26, 56 und der Magneten 27, 57, wie im Weiteren erläutert wird.

Neben der Fluidführung weisen die Wechselmodule 50A, 50B, 50C bei der vorliegenden Gestaltung auch eine datenführende Funktion auf. An der Rückseite der Wechselmodule 50A, 50B, 50C ist ein Datenkonnektor 58 vorgesehen, der im angekoppelten Zustand eines Wechselmoduls 50A, 50B, 50C an dem korrespondierenden Datenkonnektor 28 der Basiseinheit 12 anliegt.

Die Datenkommunikation zwischen der Basiseinheit 12 und den Wechselmodulen 50A, 50B, 50C kann genutzt werden, um Sensoren oder Aktoren der Wechselmodule anzusteuern. Vorliegend erfüllen sie jedoch den primären Zweck, die Verbindung der Basiseinheit 12 mit den Datenleitungen 132 zu schaffen. Die Kontaktflächen der Datenkonnektoren 58 sind hierfür über nicht näher dargestellte Signalleitungen mit Datenübertragungsmittel an den Flüssigkeitseingängen 52 und/oder Flüssigkeitsausgängen 54 verbunden. Diese Datenübertragungsmittel können insbesondere Spulen oder Kondensatorflächen zur drahtlosen Datenübertragung von Daten mit einem entsprechend angepassten Anschlussstück der Flüssigkeitsleitung 130 sein. Auch galvanisch leitfähige Kontaktflächen sind möglich.

Die Datenkonnektoren 28, 58 erlauben auf diese Weise einer Steuerungseinheit des Infusionssystems 100, mit dem Venenkatether 140 bzw. den Medikationsbeuteln 110 Daten auszutauschen.

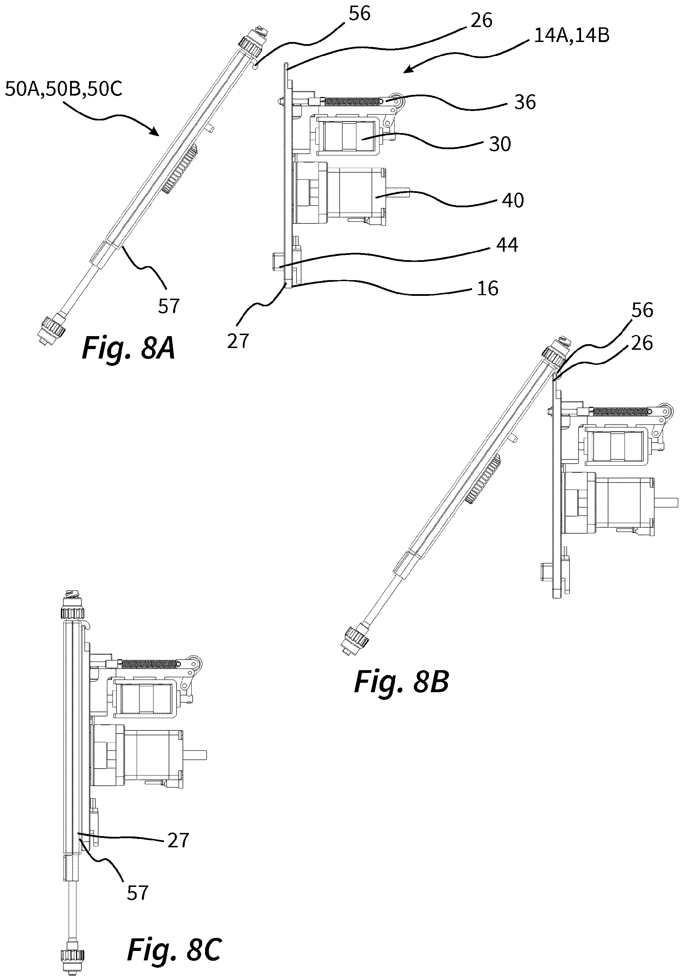

Die Figuren 8A bis 8C verdeutlichen die werkzeuglose Ankoppelung der Wechselmodule 50A, 50B, 50C an der Basiseinheit 12 bzw. an den Basismodulen 14A, 14B der Basiseinheit 12.

Die Figuren zeigen in der Seitenansicht die Basismodule 14A, 14B und deren Blende 16, an deren Rückseite der Pumpenantrieb 30, der Stellmotor 40 sowie die Ventilaktoren 36 vorgesehen sind. Wie der Darstellung der Fig. 8A zu ersehen ist, weist die Blende 16 an ihrem oberen Ende 16A eine Hängegeometrie 26 in Form einer verjüngten Abschlusskante auf.

Diese Hängegeometrie 26 ist an die hakenartigen Hängegeometrie 56 der Wechselmodule 50A angepasst, so dass in der in Fig. 8B verdeutlichten Weise das Wechselmodul 50A zunächst hier eingehängt werden kann, so dass es im folgenden schwenkverlagerbar ist.

Der Übergang von Fig. 8B nach 8C verdeutlicht den Schwenkvorgang. Beim Einschwenken fahren Ventilstifte 87 der Ventileinrichtungen in die Ventilaktoren 36 ein, so dass die Ventileinrichtung 86 über die Ventilaktoren 36 geschaltet werden können. Ein Antriebsritzel 93 der Einrichtung 92 für die Flüssigkeitsmengensteuerung gerät in Eingriff mit einem korrespondierenden Antriebsglied des Stellmotors 40. Der Druckanschluss 32 des Pumpenantriebs 30 gelangt in den Bereich der Membranwandung 74 der Pumpkammer 72, so dass über Druckwechsel am Druckanschluss 32 die Pumpkammer 72 vergrößert und verkleinert werden kann. Der an der Blende 16 exponiert nach außen angeordnete Sensor 44 gelangt in die Aussparung 81, so dass er Bläschen im Flüssigkeitskanal 60 erfassen kann.

Im heruntergeschwenkten Zustand der Fig. 8C ist das Wechselmodul 50A, 50B, 50C über die Magneten 27, 57 gesichert.

## Patentansprüche

1. Medizinische Pumpeinrichtung (10) mit den folgenden Merkmalen:
a. die Pumpeinrichtung (10) verfügt über eine Basiseinheit (12), und
b. die Pumpeinrichtung (10) verfügt über mindestens zwei flüssigkeitsleitende Wechselmodule (50A, 50B, 50C), die an einem Aufnahmebereich (22) der Basiseinheit (12) gleichzeitig ankoppelbar sind, und
c. jedes der flüssigkeitsleitenden Wechselmodule (50A, 50B, 50C) weist mindestens einen Flüssigkeitseingang (52) und mindestens einen Flüssigkeitsausgang (54) auf sowie einen vom mindestens einen Flüssigkeitseingang (52) zum Flüssigkeitsausgang (54) führenden Flüssigkeitskanal (60) auf, wobei im Flüssigkeitskanal (60) mindestens eine Pumpkammer (72) oder mindestens ein Pumpabschnitt vorgesehen ist.

2. Medizinische Pumpeinrichtung (10) nach Anspruch 1 mit dem folgenden weiteren Merkmal:
a. mindestens eines der Wechselmodule (50A, 50B) weist zwei Flüssigkeitseingänge (52) auf.

3. Medizinische Pumpeinrichtung nach Anspruch 1 oder 2 mit dem folgenden weiteren Merkmal:
a. die Basiseinheit (12) weist mindestens einen Pumpenantrieb (30) auf, mittels dessen Flüssigkeit von mindestens einem Flüssigkeitseingang (52) zum zugehörigen Flüssigkeitsausgang (54) gefördert werden kann,
vorzugsweise mit mindesten einem der folgenden zusätzlichen Merkmale:
b. mittels des Pumpenantriebs (30) ist mindestens eine Pumpkammer (72) der Wechselmodule (50A, 50B, 50C) vergrößerbar und verkleinerbar, und/oder
c. es sind mindestens zwei getrennte Pumpenantriebe (30) vorgesehen, mittels derer Flüssigkeit vom mindestens einen Flüssigkeitseingang (52) mindestens zweier Wechselmodule (50A, 50B, 50C) zum jeweiligen Flüssigkeitsausgang (54) gefördert werden kann, und/oder
d. der mindestens eine Pumpenantrieb (30) ist als pneumatischer Pumpenantrieb (30) ausgebildet, der an einem Druckanschluss (32) im Aufnahmebereich (22) alternierend einen Druckwechsel erzeugen kann, durch den die Pumpkammer (72) mindestens eines Wechselmoduls (50A, 50B, 50C) alternierend vergrößert und verkleinert wird.

4. Medizinische Pumpeinrichtung (10) nach einem der vorstehenden Ansprüche mit dem folgenden weiteren Merkmal:
a. mindestens ein Wechselmodul (50A, 50B, 50C) verfügt über eine Ventileinrichtung (86) in ihrem Flüssigkeitskanal (60),
insbesondere mit mindestens einem der folgenden zusätzlichen Merkmale:
b. mindestens eine Ventileinrichtung (86) ist zwischen dem mindestens einen Flüssigkeitseingang (52) und der Pumpkammer (72) bzw. dem Pumpabschnitt vorgesehen, wobei vorzugsweise zwischen jedem Flüssigkeitseingang (52) und der Pumpkammer (72) bzw. dem Pumpabschnitt eine Ventileinrichtung (86) vorgesehen ist, und/oder
c. mindestens eine Ventileinrichtung ist zwischen der Pumpkammer (72) bzw. dem Pumpabschnitt und dem Flüssigkeitsausgang (54) vorgesehen, und/oder
d. an der Basiseinheit (12) ist mindestens ein Ventilaktor (36) vorgesehen, mittels dessen die mindestens eine Ventileinrichtung (86) des Wechselmoduls (50A, 50B, 50C) geschlossen und geöffnet werden kann.

5. Medizinische Pumpeinrichtung (10) nach einem der vorstehenden Ansprüche mit dem folgenden weiteren Merkmal:
a. mindestens ein Wechselmodul (50A, 50B, 50C) verfügt über eine Einrichtung (92) zur Flüssigkeitsmengensteuerung,
vorzugsweise mit mindestens einem der folgenden zusätzlichen Merkmale:
b. die Einrichtung (92) zur Flüssigkeitsmengensteuerung umfasst ein variabel einstellbares Steuerventil, und/oder
c. zur Steuerung der Einrichtung (92) zur Flüssigkeitsmengensteuerung weist die Basiseinheit (12) einen Stellmotor (40) auf, der vorzugsweise als Schrittmotor gestaltet ist.

6. Medizinische Pumpeinrichtung (10) nach einem der vorstehenden Ansprüche mit den folgenden weiteren Merkmalen:
a. die Basiseinheit (12) verfügt im Aufnahmebereich (22) über mindestens zwei Daten- und/oder Stromkonnektoren (28) und die Wechselmodule (50A, 50B, 50C) verfügen jeweils über mindestens einen hierzu korrespondierenden Daten- und/oder Stromkonnektor (58), und
b. mittels der Daten- und/oder Stromkonnektoren (28, 58) können elektrische Signale zwischen der Basiseinheit (12) und den mindestens zwei Wechselmodulen (50A, 50B, 50C) ausgetauscht werden,
vorzugsweise mit mindestens einem der folgenden zusätzlichen Merkmale:
c. mindestens ein Daten- und/oder Stromkonnektor (58) eines Wechselmoduls (50A, 50B, 50C) ist mit einem in den Flüssigkeitseingang (52) und/oder in den Flüssigkeitsausgang (54) integrierten weiteren Daten- und/oder Stromkonnektor über Signalleitungen des Wechselmoduls (50A, 50B, 50C) verbunden, und/oder
d. mindestens ein Wechselmodul (50A, 50B, 50C) weist mindestens einen Sensor auf, der mit dem Datenkonnektor des Wechselmoduls verbunden ist.

7. Medizinische Pumpeinrichtung (10) nach einem der vorstehenden Ansprüche mit den folgenden weiteren Merkmalen:
a. an der Basiseinheit (12) ist mindestens ein Sensor (44) vorgesehen, der Sensordaten erzeugt, und
b. die Sensordaten des mindestens einen Sensors (44) werden durch eine Datenerfassung am Flüssigkeitskanal (60) des Wechselmoduls (50A, 50B, 50C) erzeugt,
vorzugsweise mit dem folgenden zusätzlichen Merkmal
c. der Sensor (44) ist als optischer Sensor ausgebildet, der in Richtung eines angekoppelten Wechselmoduls (50A, 50B, 50C) ausgerichtet ist, wobei vorzugsweise am Wechselmodul (50A, 50B, 50C) eine Aussparung (81) oder ein Sichtfenster im Bereich des optischen Sensors (44) vorgesehen ist.

8. Medizinische Pumpeinrichtung (10) nach einem der vorstehenden Ansprüche mit mindestens einem der folgenden Merkmale:
a. die Pumpeinrichtung (10) verfügt über mindestens drei flüssigkeitsleitende Wechselmodule (50A), die gleichzeitig an der Basiseinheit ankoppelbar sind, und/oder
b. die Pumpeinrichtung (10) verfügt über mindestens zwei baugleiche Wechselmodule (50A), und/oder
c. die Pumpeinrichtung verfügt über mindestens zwei nicht baugleiche Wechselmodule (50A, 50B, 50C), wobei diese Wechselmodule (50A, 50B, 50C) sich vorzugsweise bezüglich der Anzahl der Flüssigkeitseingänge (52) unterscheiden.

9. Medizinische Pumpeinrichtung (10) nach einem der vorstehenden Ansprüche mit dem folgenden Merkmal:
a. die Basiseinheit (12) und die Wechselmodule (50A, 50B, 50C) sind für eine werkzeuglose Kopplung und Entkopplung ausgebildet,
vorzugsweise mit mindestens einem der folgenden zusätzlichen Merkmale:
b. die Basiseinheit (12) und die Wechselmodule (50A, 50B, 50C) weisen in einem oberen Bereich des Aufnahmebereichs (22) der Basiseinheit (12) und in einem oberen Bereich einer Rückwandung der Wechselmodule (50A, 50B, 50C) zusammenwirkende Hängegeometrien (56, 26) auf, und/oder
c. die Basiseinheit (12) und die Wechselmodule (50A, 50B, 50C) weisen zusammenwirkende Schnappgeometrien auf, mittels derer die Wechselmodule an der Basiseinheit festschnappbar sind.

10. Medizinische Pumpeinrichtung (10) nach einem der vorstehenden Ansprüche mit dem folgenden weiteren Merkmal:
a. die Basiseinheit (12) weist eine Mehrzahl von Basismodulen (14A, 14B) auf, die separat austauschbar sind und deren Vorderseite den Aufnahmebereich (22) zur Aufnahme der Wechselmodule (50A, 50B, 50C) begrenzt.
insbesondere mit mindestens einem der folgenden zusätzlichen Merkmale:
b. es sind mindestens zwei Basismodule (14A, 14B) vorgesehen, die
- jeweils über einen Pumpenantrieb (30) und/oder
- jeweils über mindestens einen Ventilaktor (36) zur Steuerung einerVentileinrichtung (86) eines Wechselmoduls () und/oder
- jeweils über mindestens einen Stellmotor (40) zur Steuerung einer Einrichtung (92) zur Flüssigkeitsmengensteuerung eines Wechselmoduls (50A, 50B, 50C).
verfügen.

11. Medizinische Pumpeinrichtung (10) nach Anspruch 10 mit dem folgenden weiteren Merkmal:
a. die Basismodule (14A, 14B) sind als nicht werkzeuglos austauschbare Module der Basiseinheit (12) ausgebildet.

12. Medizinische Pumpeinrichtung (10) nach einem der vorstehenden Ansprüche mit dem folgenden weiteren Merkmal:
a. mindestens ein Wechselmodul weist einen integrierten Schaltkreis und/oder einen elektronischen Speicher umfassen,
vorzugsweise mit mindestens einem der folgenden weiteren Merkmale:
b. der elektronische Speicher beinhaltet eine Typinformationen über das Wechselmodul, und/oder
c. der elektronische Speicher beinhaltet eindeutige Kennzeichnung des Wechselmoduls, und/oder
d. der elektronische Speicher beinhaltet Spezifikationen des Wechselmoduls, insbesondere zu den vorgesehenen Funktionen und Volumina, und/oder
e. der elektronische Speicher beinhaltet eine Benutzungshistorie und/oder Haltbarkeitsdaten.

13. Medizinische Pumpeinrichtung (10) nach einem der vorstehenden Ansprüche mit mindestens einem der folgenden Merkmale:
a. die Basiseinheit (12) weist keine flüssigkeitsführenden Kanäle, Kammern oder Flächen auf, und/oder
b. die Basiseinheit (12) verfügt über mindestens drei Pumpenantriebe (30), und/oder
c. mindestens ein Wechselmodul (50A, 50B, 50C) ist mit einer Pumpkammer (72) ausgestattet, die auf einer Seite durch eine formflexible oder verlagerbare Wandung (74) begrenzt ist, durch den Druckwechsel am Druckanschluss (32) verformt bzw. verlagerbar ist, und/oder
d. die Wechselmodule (50A, 50B, 50C) sind als Wechselkassetten mit einem Außengehäuse aus Kunststoff ausgestaltet, und/oder
e. mindestens ein Flüssigkeitseingang (52) und mindestens ein Flüssigkeitsausgang (54) sind auf einander gegenüberliegenden Seite des Wechselmodus (50A, 50B, 50C) vorgesehen.

14. Infusionssystem (100) mit dem folgenden Merkmal:
a. das Infusionssystem (100) umfasst eine Pumpeinrichtung (10) zur Förderung von medizinischen Flüssigkeiten aus Medikationsbehältern (110) zu einem Patienten (120),
**gekennzeichnet durch** das folgende zusätzliche Merkmal:
b. die Pumpeinrichtung (10) ist nach einem der vorstehenden Ansprüche ausgebildet.
